# EUROPÄISCHE PATENTANMELDUNG

(11) **EP 2 626 061 A2**
(43) Veröffentlichungstag der Anmeldung: **14.08.2013**
(21) Anmeldenummer: 12195575.1
(22) Anmeldetag: 05.12.2012
(51) Int. Cl.: A61K 8/26, A61K 8/31, A61K 8/37, A61K 8/41, A61K 8/49, A61K 8/58, A61Q 15/00, A61K 8/04

(54) **Verfahren zur Herstellung eines kosmetischen Produkts mit einer verdickten Ölphase**

(30) Priorität: 22.12.2011 DE 102011089610
(71) Anmelder: Henkel AG&Co. KGAA, 40589 Düsseldorf (DE)
(72) Erfinder: Banowski, Bernhard, 40597 Düsseldorf (DE); Teckenbrock, Gertraud, 45549 Sprockhövel (DE); Mausberg, Sandra, 42781 Haan (DE); Solich, Daniel, 40764 Langenfeld (DE)

(57) **Zusammenfassung**

Gegenstand der vorliegenden Anmeldung ist ein Verfahren zur Herstellung eines kosmetischen Produkts, das eine kosmetische Zusammensetzung mit einer verdickten Ölphase und einen Verpackungsbehälter umfasst.

## Beschreibung

Gegenstand der vorliegenden Anmeldung ist ein Verfahren zur Herstellung eines kosmetischen Produkts, das eine kosmetische Zusammensetzung mit einer verdickten Ölphase und einen Verpackungsbehälter umfasst.

Sprayprodukte sind aus dem modernen Alltagsleben kaum noch wegzudenken. Zahlreiche kosmetische und pharmazeutische Produkte, wie Haarsprays, Deosprays und Mundsprays, Produkte zur Haushaltspflege, wie Backofenreinigungssprays, Bügelhilfesprays oder Raumsprays, sowie weiterhin Produkte zur Auto- oder Lackpflege ermöglichen durch die Sprayapplikation einen bequemen, schnellen und gleichmäßigen Produktauftrag, der von vielen Verbrauchern sehr geschätzt wird. Eine beim Verbraucher besonders beliebte Produktform sind Antitranspirantsprays. Diese enthalten ein schweißhemmendes Aluminiumsalz, das im Kontakt mit Wasser relativ sauer reagiert. Aus diesem Grund sind wasserhaltige Antitranspirantsprays eher ein Nischenprodukt, denn die Entwicklung einer stabilen, nicht-korrosiven und wirksamen Rezeptur ist nicht trivial. Demgegenüber erfreuen sich wasserfreie Antitranspirantsprays großer Beliebtheit im Markt. Bei dieser Produktform liegt das schweißhemmende Aluminiumsalz, das laut gesetzlicher Vorschrift Zirconium-frei sein muss, in mindestens einem unter Normalbedingungen flüssigen Öl suspendiert vor. Zur besseren Anwendbarkeit wird dieser Suspension noch mindestens ein lipophiles Verdickungsmittel als Suspendierhilfe zugesetzt.

Als lipophile Verdickungsmittel sind prinzipiell zahlreiche Substanzen geeignet, insbesondere Schichtsilikate, vor allem hydrophob modifizierte Schichtsilikate, weiterhin pyrogene Kieselsäuren, Ethylene/Propylene/Styrene Copolymere, Butylene/Ethylene/Styrene Copolymere, Dextrinester und Siliconelastomere. Hierunter sind Schichtsilikate, insbesondere hydrophob modifizierte Schichtsilikate, besonders bevorzugt, denn sie verleihen der verdickten Ölphase zum Versprühen geeignete rheologische Eigenschaften, sind preisgünstig verfügbar und weisen insgesamt gute kosmetische Eigenschaften auf.

Um ein wasserfreies Antitranspirantspray, wie vorstehend geschildert, herzustellen, war es bislang üblich, die Bestandteile der Ölphase bei Raumtemperatur mit mindestens einem Schichtsilikat, vor allem mit mindestens einem hydrophob modifizierten Schichtsilikat, zu versetzen, die Gelbildung durch den Zusatz geringer Mengen eines polaren Aktivators, wie insbesondere Ethanol oder Propylencarbonat, aber auch Wasser, zu starten, anschließend das pulverförmige schweißhemmende Aluminiumsalz zuzusetzen, die Suspension in eine Aerosol-Abgabevorrichtung abzufüllen und mit einem Treibmittel zu versehen.

Ein Nachteil der bekannten Produkte ist, dass sie auf der Haut und der mit der Haut in Kontakt kommenden Kleidung sichtbare Rückstände hinterlassen, die insbesondere vom ungelösten Aluminiumsalz, aber auch vom Schichtsilikat-Verdicker, herrühren.

Aufgabe der vorliegenden Erfindung war es, ein bekanntes Herstellverfahren für kosmetische Produkte, die eine verdickte Ölphase enthalten, weiter zu optimieren, um ein qualitativ höherwertiges Produkt zu erhalten, das weniger sichtbare Rückstände hinterlässt. Daneben bestand die Aufgabe, die Kosten der Herstellung, beispielsweise durch eingesparte Rohstoffkosten, zu senken. Daneben bestand die Aufgabe, das Herstellverfahren zu vereinfachen. Daneben bestand die Aufgabe, ein stabileres Produkt mit konstanterer Spezifikation zu erzielen.

Überraschend wurde festgestellt, dass die gestellten Aufgaben sehr gut gelöst wurden, indem die Gelbildung mit einer erwärmten Ölphase durchgeführt wurde. Gegenüber dem konventionellen Herstellverfahren bei Raumtemperatur, das heißt insbesondere, bei 10 - 25 °C, bevorzugt 14 - 22 °C, wurde eine effizientere Verdickung erzielt, das heißt, die für ein optimales Sprayprodukt erforderliche Viskosität konnte mit weniger Schichtsilikat und weniger polarem Aktivator erzielt werden als bisher. Das so erhaltene Produkt hinterließ auf der Haut und auf Textilien weniger sichtbare Rückstände als das bisherige Produkt. Durch die Reduktion der Rohstoffmenge wurde eine deutliche Kostenersparnis erzielt, die die Steigerung der Energiekosten des neuen Verfahrens überkompensierte. Die Endviskosität stellte sich schneller ein als bisher, so dass die verdickte Ölphase schneller als bisher zum Endprodukt weiterverarbeitet werden konnte. Zwischen verschiedenen Produktionschargen wurden geringere Abweichungen der Endviskosität beobachtet, die erzielte Endviskosität war, über mehrere Chargen gemittelt, konstanter.

Gegenstand der vorliegenden Erfindung ist daher ein Verfahren zur Herstellung eines kosmetischen Produkts, das eine kosmetische Zusammensetzung mit einer verdickten Ölphase und einen Verpackungsbehälter umfasst, wobei das Verfahren folgende Verfahrensschrittfolge aufweist:
I) Bereitstellen von mindestens einem Öl oder mindestens einer Ölmischung, das bzw. die unter Normalbedingungen (20 °C, 1013,25 mbar) flüssig ist,
II) Erwärmen des Öls bzw. der Ölmischung auf eine Temperatur von 30 - 55 °C, bevorzugt 30 - 35 °C,
III) Zugabe von mindestens einem Schichtsilikat und mindestens einem polaren Aktivator,
IV) Homogenisieren, bevorzugt mit einer Scherrate von 500 - 2000 Upm (Umdrehungen pro Minute),
V) Abkühlen auf eine Temperatur im Bereich von 10 bis < 30 °C, bevorzugt im Bereich von 14 bis 22 °C,
VI) Zugabe von mindestens einem weiteren kosmetischen Wirk- und/oder Hilfsstoff,
VII) Abfüllen in einen Verpackungsbehälter.

Bei allen Mengenangaben in dieser Anmeldung bleibt das Gewicht der Aerosol-Abgabevorrichtung stets unberücksichtigt.

"Normalbedingungen" sind im Sinne der vorliegenden Anmeldung eine Temperatur von 20 °C und ein Druck von 1013,25 mbar. Schmelzpunktangaben beziehen sich ebenfalls auf einen Druck von 1013,25 mbar.

Die Gesamtmenge an unter Normalbedingungen flüssigen kosmetischen Ölen beträgt in erfindungsgemäß bevorzugten Zusammensetzungen 1 - 95 Gew.-%, bevorzugt 5 - 90 Gew.-%, besonders bevorzugt 30 - 75 Gew.-%, außerordentlich bevorzugt 50 - 60 Gew.-%, wobei sich die Mengenangaben auf das Gewicht der Zusammensetzung beziehen, ohne das Gewicht des Treibmittels zu berücksichtigen.

Das kosmetische Öl ist unter Normalbedingungen flüssig; ätherische Öle und Parfümöle bzw. Riechstoffe werden nicht zu den kosmetischen Ölen gezählt. Die unter Normalbedingungen flüssigen kosmetischen Öle sind mit Wasser nicht mischbar. Unter ätherischen Ölen werden erfindungsgemäß Gemische aus flüchtigen Komponenten verstanden, die durch Wasserdampfdestillation aus pflanzlichen Rohstoffen hergestellt werden, wie z. B. Citrusöle. Sofern in der vorliegenden Anmeldung von einem kosmetischen Öl die Rede ist, handelt es sich hierbei immer um ein kosmetisches Öl, das kein Riechstoff und kein ätherisches Öl ist, unter Normalbedingungen flüssig und mit Wasser nicht mischbar ist.

Die Definition eines Riechstoffs im Sinne der vorliegenden Anmeldung stimmt überein mit der fachmännisch üblichen Definition, wie sie dem RÖMPP Chemie Lexikon, Stand Dezember 2007, entnommen werden kann. Danach ist ein Riechstoff eine chemische Verbindung mit Geruch und/oder Geschmack, der die Rezeptoren der Haarzellen des olfaktorischen Systems erregt (adäquater Reiz). Die hierzu notwendigen physikalischen und chemischen Eigenschaften sind eine niedrige Molmasse von maximal 300 g/mol, ein hoher Dampfdruck, minimale Wasser- und hohe Lipidlöslichkeit sowie schwache Polarität und das Vorliegen mindestens einer osmophoren Gruppe im Molekül. Um flüchtige, niedermolekulare Substanzen, die üblicherweise und auch im Sinne der vorliegenden Anmeldung nicht als Riechstoff, sondern vornehmlich als Lösemittel angesehen und verwendet werden, wie beispielsweise Ethanol, Propanol, Isopropanol und Aceton, von erfindungsgemäßen Riechstoffen abzugrenzen, weisen erfindungsgemäße Riechstoffe eine Molmasse von 74 bis 300 g/mol auf, enthalten mindestens eine osmophore Gruppe im Molekül und weisen einen Geruch und/oder Geschmack auf, das heißt, sie erregen die Rezeptoren der Haarzellen des olfaktorischen Systems.

Die Gesamtmenge an unter Normalbedingungen flüssigen kosmetischen Ölen beträgt in erfindungsgemäß bevorzugt hergestellten Antitranspirant-Produkten 1 - 95 Gew.-%, bevorzugt 5 - 90 Gew.-%, besonders bevorzugt 10 - 85 Gew.-%, außerordentlich bevorzugt 20 - 80 Gew.-%, weiter bevorzugt 30 - 75 Gew.-%, ebenfalls sehr bevorzugt 50 - 70 Gew.-%, ebenfalls außerordentlich bevorzugt 54 - 64 Gew.-%, wobei sich die Mengenangaben auf das Gewicht der Zusammensetzung beziehen, ohne das Gewicht des Treibmittels zu berücksichtigen. Auch eine Gesamtmenge an mindestens einem unter Normalbedingungen flüssigen kosmetischen Öl von 60 - 80 Gew.-% und 65 - 76 Gew.-%, jeweils bezogen auf die gesamte treibmittelfreie Zusammensetzung, kann erfindungsgemäß besonders bevorzugt sein, wobei eine Gesamtmenge von 69 - 74 Gew.-%, bezogen auf die gesamte treibmittelfreie Zusammensetzung, besonders bevorzugt ist.

Bei den kosmetischen Ölen unterscheidet man flüchtige und nicht-flüchtige Öle. Unter nicht-flüchtigen Ölen versteht man solche Öle, die bei 20 °C und einem Umgebungsdruck von 1013 hPa einen Dampfdruck von weniger als 2,66 Pa (0,02 mm Hg) aufweisen. Unter flüchtigen Ölen versteht man solche Öle, die bei 20 °C und einem Umgebungsdruck von 1013 hPa einen Dampfdruck von 2,66 Pa - 40000 Pa (0,02 mm - 300 mm Hg), bevorzugt 13 - 12000 Pa (0,1 - 90 mm Hg), besonders bevorzugt 14 - 3000 Pa, außerordentlich bevorzugt 15 - 500 Pa, aufweisen. Flüchtige kosmetische Öle sind üblicherweise unter cyclischen Siliconölen mit der INCI-Bezeichnung Cyclomethicone ausgewählt. Unter der INCl-Bezeichnung Cyclomethicone werden insbesondere Cyclotrisiloxan (Hexamethylcyclotrisiloxan), Cyclotetrasiloxan (Octamethylcyclotetrasiloxan), Cyclopentasiloxan (Decamethylcyclopentasiloxan) und Cyclohexasiloxan (Dodecamethylcyclohexasiloxan) verstanden. Diese Öle weisen bei 20°C einen Dampfdruck von ca. 13 - 15 Pa auf.

Cyclomethicone sind im Stand der Technik als gut geeignete Öle für kosmetische Produkte, insbesondere für schweißhemmende Produkte bekannt. Aufgrund ihrer Persistenz in der Umwelt kann es aber erfindungsgemäß bevorzugt sein, auf den Einsatz von Cyclomethiconen zu verzichten. In einer speziell bevorzugten Ausführungsform enthalten die erfindungsgemäßen Zusammensetzungen 0 bis weniger als 1 Gew.-% Cyclomethicone, bezogen auf das Gewicht der Zusammensetzung, ohne das Gewicht des Treibmittels zu berücksichtigen.

Ein bevorzugter Cyclomethicone-Ersatzstoff ist eine Mischung aus C13-C16 Isoparaffinen, C12 - C14 Isoparaffinen und C13 - C15 Alkanen, deren Viskosität bei 25°C im Bereich von 2 bis 6 mPas liegt und die einen Dampfdruck bei 20°C im Bereich von 100 bis 150 Pa aufweist. Eine solche Mischung ist z. B. unter der Bezeichnung SiClone SR-5 von der Firma Presperse Inc. erhältlich.

Weitere bevorzugte flüchtige Siliconöle sind ausgewählt aus flüchtigen linearen Siliconölen, insbesondere flüchtigen linearen Siliconölen mit 2 - 10 Siloxaneinheiten, wie Hexamethyldisiloxan (L₂), Octamethyltrisiloxan (L₃), Decamethyltetrasiloxan (L₄), wie sie z. B. in den Handelsprodukten DC 2-1184, Dow Corning^{®} 200 (0,65 cSt) und Dow Corning^{®} 200 (1,5 cSt) von Dow Corning enthalten sind, und niedermolekulares Phenyl Trimethicone mit einem Dampfdruck bei 20°C von etwa 2000 Pa, wie es beispielsweise von GE Bayer Silicones/Momentive unter dem Namen Baysilone Fluid PD 5 erhältlich ist.

Bevorzugte erfindungsgemäße Zusammensetzungen, insbesondere Antitranspirant-Zusammensetzungen, enthalten wegen des trockeneren Hautgefühls und der schnelleren Wirkstofffreisetzung mindestens ein flüchtiges Siliconöl, das cyclisch oder linear sein kann.

Weitere bevorzugte erfindungsgemäß hergestellte Zusammensetzungen, insbesondere Antitranspirant-Zusammensetzungen, enthalten wegen des trockeneren Hautgefühls und der schnelleren Wirkstofffreisetzung mindestens ein flüchtiges Nichtsiliconöl. Bevorzugte flüchtige Nichtsiliconöle sind ausgewählt aus C₈-C₁₆-Isoparaffinen, insbesondere aus Isononan, Isodecan, Isoundecan, Isododecan, Isotridecan, Isotetradecan, Isopentadecan, und Isohexadecan, sowie Mischungen hiervon. Bevorzugt sind C₁₀-C₁₃-Isoparaffin-Mischungen, insbesondere solche mit einem Dampfdruck bei 20°C von 10 - 400 Pa, bevorzugt 13 - 100 Pa. Dieses mindestens eine C₈-C₁₆-Isoparaffin ist bevorzugt in einer Gesamtmenge von 25 - 50 Gew.-%, bevorzugt 30 - 45 Gew.-%, besonders bevorzugt 32 - 40 Gew.-%, außerordentlich bevorzugt 34 - 37 Gew.-%, jeweils bezogen auf die gesamte treibmittelfreie Zusammensetzung, enthalten.

In erfindungsgemäß bevorzugt hergestellten Produkten umfasst das mindestens eine unter Normalbedingungen flüssige Öl mindestens ein flüchtiges C₈-C₁₆-Isoparaffin, insbesondere Isononan, Isodecan, Isoundecan, Isododecan, Isotridecan, Isotetradecan, Isopentadecan und Isohexadecan sowie Mischungen hiervon.

Weitere erfindungsgemäß bevorzugt hergestellte Produkte enthalten Triethylcitrat und mindestens ein C₈-C₁₆-Isoparaffin, ausgewählt aus Isononan, Isodecan, Isoundecan, Isododecan, Isotridecan, Isotetradecan, Isopentadecan und Isohexadecan sowie Mischungen dieser Isoparaffine.

Weitere erfindungsgemäß bevorzugt hergestellte Produkte enthalten Triethylcitrat und mindestens ein C₈-C₁₆-Isoparaffin, ausgewählt aus Isononan, Isodecan, Isoundecan, Isododecan, Isotridecan sowie Mischungen dieser C₈-C₁₆-Isoparaffine.

Weitere erfindungsgemäß bevorzugt hergestellte Produkte enthalten Triethylcitrat und eine Mischung aus Isodecan, Isoundecan, Isododecan und Isotridecan.

Weitere erfindungsgemäß bevorzugt hergestellte Produkte, insbesondere Antitranspirant-Zusammensetzungen, enthalten mindestens ein nichtflüchtiges kosmetisches Öl, ausgewählt aus nichtflüchtigen Siliconölen und nichtflüchtigen Nichtsiliconölen. Rückstände von im Träger unlöslichen Bestandteilen, wie Antitranspirantwirkstoffe oder Talkum, können erfolgreich mit einem nichtflüchtigen Öl maskiert werden. Außerdem können mit einem Gemisch aus verschiedenen Ölen, insbesondere aus nichtflüchtigem und flüchtigem Öl, Parameter wie Hautgefühl, Sichtbarkeit des Rückstands und Stabilität der erfindungsgemäßen Zusammensetzung feinreguliert und besser an die Bedürfnisse der Verbraucher angepasst werden.

Selbstverständlich ist es ebenfalls möglich, erfindungsgemäße Produkte mit einem geringen Anteil an flüchtigen Ölen - das heißt, mit 0,5 - 24,5 Gew.-% an flüchtigen Ölen, bezogen auf das Gewicht der treibmittelfreien Zusammensetzung - oder sogar ohne flüchtige Öle zu formulieren. Erfindungsgemäß besonders bevorzugt sind Ester der linearen oder verzweigten gesättigten oder ungesättigten Fettalkohole mit 2 - 30 Kohlenstoffatomen mit linearen oder verzweigten gesättigten oder ungesättigten Fettsäuren mit 2 - 30 Kohlenstoffatomen, die hydroxyliert sein können. Bevorzugt sind Ester der linearen oder verzweigten gesättigten Fettalkohole mit 2 - 5 Kohlenstoffatomen mit linearen oder verzweigten gesättigten oder ungesättigten Fettsäuren mit 10 - 18 Kohlenstoffatomen, die hydroxyliert sein können. Bevorzugte Beispiele hierfür sind Isopropylpalmitat, Isopropylstearat, Isopropylmyristat, 2-Hexyldecylstearat, 2-Hexyldecyllaurat, Isononylisononanoat, 2-Ethylhexylpalmitat und 2-Ethylhexylstearat. Ebenfalls bevorzugt sind Isooctylstearat, Isononylstearat, Isocetylstearat, Isononylisononanoat, Isotridecylisononanoat, Cetearylisononanoat, 2-Ethylhexyllaurat, 2-Ethylhexylisostearat, 2-Ethylhexylcocoat, 2-Octyldodecylpalmitat, Butyl-octansäure-2-butyloctanoat, Diisotridecylacetat, n-Hexyllaurat, n-Decyloleat, Oleyloleat, Oleylerucat, Erucyloleat, C₁₂-C₁₅-Alkyllactat und Di-C₁₂-C₁₃-Alkylmalat sowie die Benzoesäureester von linearen oder verzweigten C₈₋₂₂-Alkanolen. Besonders bevorzugt sind Benzoesäure-C₁₂-C₁₅-Alkylester, z. B. erhältlich als Handelsprodukt Finsolv^{®} TN (C₁₂-C₁₅-Alkylbenzoat), sowie Benzoesäureisostearylester, z. B. erhältlich als Finsolv^{®} SB, 2-Ethylhexylbenzoat, z. B. erhältlich als Finsolv^{®} EB, und Benzoesäure-2-octyldocecylester, z. B. erhältlich als Finsolv^{®} BOD. Ein weiteres besonders bevorzugtes Esteröl ist Triethylcitrat.

Erfindungsgemäß bevorzugte Ölmischungen sind Triethylcitrat/2-Ethylhexylpalmitat, Triethylcitrat/Hexyldecyllaurat, Triethylcitrat/2-Ethylhexylstearat, Triethylcitrat/Isopropylmyristat, Triethylcitrat/Isopropylpalmitat, Triethylcitrat/2-Ethylhexyllaurat, Triethylcitrat/C₁₂-C₁₅-Alkyllactat, Triethylcitrat/C₁₂-C₁₅-Alkylbenzoat und Triethylcitrat/Di-C₁₂-C₁₃-Alkylmalat. Besonders bevorzugte Ölmischungen sind Triethylcitrat/Isopropylmyristat, Triethylcitrat/Isopropylpalmitat, Triethylcitrat/2-Ethylhexylpalmitat und Triethylcitrat/C₁₂-C₁₅-Alkylbenzoat.

Weitere erfindungsgemäß bevorzugte nichtflüchtige Nichtsiliconöle sind ausgewählt aus verzweigten gesättigten oder ungesättigten Fettalkoholen mit 6 - 30 Kohlenstoffatomen. Diese Alkohole werden häufig auch als Guerbet-Alkohole bezeichnet, da sie nach der Guerbet-Reaktion erhältlich sind. Bevorzugte Alkoholöle sind 2-Hexyldecanol, 2-Octyldodecanol und 2-Ethylhexylalkohol. Ebenfalls bevorzugt ist Isostearylalkohol. Weitere bevorzugte nichtflüchtige Öle sind ausgewählt aus Mischungen aus Guerbetalkoholen und Guerbetalkoholestern, z. B. 2-Hexyldecanol und 2-Hexyldecyllaurat.

Der im folgenden gebrauchte Ausdruck "Triglycerid" meint "Glycerintriester". Weitere erfindungsgemäß bevorzugte nichtflüchtige Öle sind ausgewählt aus den Triglyceriden von linearen oder verzweigten, gesättigten oder ungesättigten, gegebenenfalls hydroxylierten C₈₋₃₀-Fettsäuren, sofern diese unter Normalbedingungen flüssig sind. Besonders geeignet kann die Verwendung natürlicher Öle, z.B. Sojaöl, Baumwollsaatöl, Sonnenblumenöl, Palmöl, Palmkernöl, Leinöl, Mandelöl, Rizinusöl, Maisöl, Rapsöl, Olivenöl, Sesamöl, Distelöl, Weizenkeimöl, Pfirsichkernöl und die flüssigen Anteile des Kokosöls und dergleichen sein. Besonders bevorzugt sind synthetische Triglyceridöle, insbesondere Capric/Caprylic Triglycerides, z. B. die Handelsprodukte Myritol^{®} 318 oder Myritol^{®} 331 (BASF/ Cognis) mit unverzweigten Fettsäureresten sowie Glyceryltriisostearin und Glyceryltri(2-ethylhexanoat) mit verzweigten Fettsäureresten. Derartige Triglyceridöle machen bevorzugt einen Anteil von weniger als 50 Gew.-% am Gesamtgewicht aller kosmetischen Öle in dem erfindungsgemäßen Produkt aus. Besonders bevorzugt beträgt das Gesamtgewicht an Triglyceridölen 0,5 - 10 Gew.-%, bevorzugt 1 - 5 Gew.-%, jeweils bezogen auf die gesamte Zusammensetzung, ohne das Gewicht des Treibmittels zu berücksichtigen.

Weitere erfindungsgemäß besonders bevorzugte nichtflüchtige Nichtsiliconöle sind ausgewählt aus den Dicarbonsäureestern von linearen oder verzweigten C₂-C₁₀-Alkanolen, insbesondere Diisopropyladipat, Di-n-butyladipat, Di-(2-ethylhexyl)adipat, Dioctyladipat, Diethyl-/Di-n-butyl/Dioctylsebacat, Diisopropylsebacat, Dioctylmalat, Dioctylmaleat, Dicaprylylmaleat, Diisooctylsuccinat, Di-2-ethylhexylsuccinat und Di-(2-hexyldecyl)-succinat.

Weitere erfindungsgemäß besonders bevorzugte nichtflüchtige Nichtsiliconöle sind ausgewählt aus den Anlagerungsprodukten von 1 bis 5 Propylenoxid-Einheiten an ein- oder mehrwertige C₈-₂₂-Alkanole wie Octanol, Decanol, Decandiol, Laurylalkohol, Myristylalkohol und Stearylalkohol, z. B. PPG-2-Myristylether und PPG-3-Myristylether.

Weitere erfindungsgemäß besonders bevorzugte nichtflüchtige Nichtsiliconöle sind ausgewählt aus den Anlagerungsprodukten von mindestens 6 Ethylenoxid- und/oder Propylenoxid-Einheiten an ein- oder mehrwertige C₃₋₂₂-Alkanole wie Glycerin, Butanol, Butandiol, Myristylalkohol und Stearylalkohol, die gewünschtenfalls verestert sein können, z. B. PPG-14-Butylether, PPG-9-Butylether, PPG-10-Butandiol, PPG-15-Stearylether und Glycereth-7-diisononanoat.

Weitere erfindungsgemäß besonders bevorzugte nichtflüchtige Nichtsiliconöle sind ausgewählt aus den symmetrischen, unsymmetrischen oder cyclischen Estern der Kohlensäure mit C₆-C₂₀-Alkoholen, z. B. Di-n-caprylylcarbonat (Cetiol^{®} CC) oder Di-(2-ethylhexyl)carbonat (Tegosoft DEC). Ester der Kohlensäure mit C₁-C₅-Alkoholen, z. B. Glycerincarbonat oder Propylencarbonat, sind hingegen keine als kosmetisches Öl geeigneten Verbindungen. Propylencarbonat kann in den erfindungsgemäßen Zusammensetzungen gleichwohl enthalten sein, vornehmlich als Aktivator für das lipophile Verdickungsmittel, insbesondere für hydrophob modifizierte Bentonite und Hectorite. Propylencarbonat und andere Ester der Kohlensäure mit C₁-C₅-Alkoholen nicht bei der Berechnung der Gesamtmenge an Ölen sind zu berücksichtigen.

Weitere Öle, die erfindungsgemäß bevorzugt sein können, sind ausgewählt aus den Estern von Dimeren ungesättigter C₁₂-C₂₂-Fettsäuren (Dimerfettsäuren) mit einwertigen linearen, verzweigten oder cyclischen C₂-C₁₈-Alkanolen oder mit mehrwertigen linearen oder verzweigten C₂-C₆-Alkanolen. Besonders bevorzugt beträgt das Gesamtgewicht an Dimerfettsäureestern 0,5 - 10 Gew.- %, bevorzugt 1 - 5 Gew.-%, jeweils bezogen auf die gesamte Zusammensetzung, ohne das Gewicht des Treibmittels zu berücksichtigen.

Ein weiteres erfindungsgemäß bevorzugtes Öl ist Triethylcitrat. Besonders bevorzugte Öle bestehen aus Mischungen der vorgenannten Öle. Weiterhin ist es besonders bevorzugt, Mischungen aus Triethylcitrat und mindestens einem Ester der linearen oder verzweigten gesättigten oder ungesättigten Fettalkohole mit 2 - 30 Kohlenstoffatomen mit linearen oder verzweigten gesättigten oder ungesättigten Fettsäuren mit 2 - 30 Kohlenstoffatomen, die hydroxyliert sein können, zu verwenden. In derartigen Mischungen sind die Ethylester und die Isopropylester besonders bevorzugt; außerordentlich bevorzugt sind Isopropylpalmitat und Isopropylmyristat. Eine weitere erfindungsgemäß besonders bevorzugte Ölmischung ist Triethylcitrat/2-Ethylhexylpalmitat. Bevorzugte erfindungsgemäße Verfahren sind dadurch gekennzeichnet, dass das mindestens eine unter Normalbedingungen flüssige kosmetische Öl ausgewählt ist aus cyclischen Siliconölen mit der INCI-Bezeichnung Cyclomethicone, flüchtigen linearen Siliconölen, insbesondere flüchtigen linearen Siliconölen mit 2 - 10 Siloxan-Einheiten, C₈-C₁₆-Isoparaffinen, C₁₀-C₁₃-lsoparaflinmischungen, Estern der linearen oder verzweigten gesättigten oder ungesättigten Fettalkohole mit 2 - 30 Kohlenstoffatomen mit linearen oder verzweigten gesättigten oder ungesättigten Fettsäuren mit 2 - 30 Kohlenstoffatomen, die hydroxyliert sein können, Benzoesäureestern von linearen oder verzweigten C₈-C₂₂-Alkanolen, Triethylcitrat, verzweigten gesättigten oder ungesättigten Fettalkoholen mit 6 - 30 Kohlenstoffatomen, den Triglyceriden von linearen oder verzweigten, gesättigten oder ungesättigten, gegebenenfalls hydroxylierten C₈₋₃₀-Fettsäuren, sofern diese unter Normalbedingungen flüssig sind, Dicarbonsäureestern von linearen oder verzweigten C₂-C₁₀-Alkanolen, Anlagerungsprodukten von 1 bis 5 Propylenoxid-Einheiten an ein- oder mehrwertige C₈₋₂₂-Alkanole, Anlagerungsprodukten von mindestens 6 Ethylenoxid- und/oder Propylenoxid-Einheiten an ein- oder mehrwertige C₃₋₂₂-Alkanole, den symmetrischen, unsymmetrischen oder cyclischen Estern der Kohlensäure mit C₆-C₂₀-Alkoholen und den Estern von Dimeren ungesättigter C₁₂-C₂₂-Fettsäuren (Dimerfettsäuren) mit einwertigen linearen, verzweigten oder cyclischen C₂-C₁₈-Alkanolen oder mit mehrwertigen linearen oder verzweigten C₂-C₆-Alkanolen, sowie Mischungen hiervon.

Ein erfindungsgemäß bevorzugtes Verfahren ist dadurch gekennzeichnet, dass das Schichtsilikat ausgewählt ist aus Hectorit, Bentonit, Montmorrillonit, Nontronit, Saponit, Sauconit, Beidellit, Allevardit, Illit, Halloysit, Attapulgit und/oder Sepiolit, sowie Mischungen hiervon. Besonders bevorzugt sind Hectorit und Bentonit, sowie Mischungen hiervon.

Ein erfindungsgemäß besonders bevorzugtes Verfahren ist dadurch gekennzeichnet, dass das Schichtsilikat, bevorzugt ausgewählt aus Hectorit und Bentonit, mit mindestens einer organischen kationischen Substanz hydrophob modifiziert vorliegt.

Ein erfindungsgemäß außerordentlich bevorzugtes Verfahren ist dadurch gekennzeichnet, dass das mindestens eine hydrophob modifizierte Schichtsilikat ausgewählt ist aus Disteardimonium Hectorite, Stearalkonium Hectorite, Stearalkonium Bentonite, Quaternium-18 Hectorite, Quaternium-18 Bentonite und Dihydrogenated Tallow Benzylmonium Hectorite, sowie Mischungen hiervon.

Ein weiteres erfindungsgemäß außerordentlich bevorzugtes Verfahren ist dadurch gekennzeichnet, dass die treibmittelfreie Zusammensetzung mindestens ein hydrophob modifiziertes Schichtsilikat, das in Verfahrensschritt III zugesetzt wird, in einer Gesamtmenge von 0,5 - 10 Gew.-%, bevorzugt 1 - 7 Gew.-%, besonders bevorzugt 2 - 6 Gew.-%, außerordentlich bevorzugt 3 - 5 Gew.-%, enthält, jeweils bezogen auf das Gesamtgewicht der treibmittelfreien Zusammensetzung. Die Gewichtsangaben beziehen sich bei chemisch modifizierten Schichtsilikaten immer auf das Gewicht des Schichtsilikats inclusive des Modifikationsmittels.

Ein weiteres erfindungsgemäß außerordentlich bevorzugtes Verfahren ist dadurch gekennzeichnet, dass das Gewichtsverhältnis von Gesamtgewicht an Öl zum Gesamtgewicht an ggf. hydrophob modifiziertem Schichtsilikat 12 - 45, bevorzugt 15 - 35, besonders bevorzugt 20 - 30, beträgt. Für die Gelbildung durch das Schichtsilikat, das bevorzugt hydrophob modifiziert ist, benötigt man üblicherweise als polaren Aktivator Wasser, Ethanol oder Propylencarbonat. Der polare Aktivator ist bevorzugt in einer Menge von 0,3 - 3 Gew.-%, bevorzugt 0,5 - 2 Gew.-%, jeweils bezogen auf das Gesamtgewicht der treibmittelfreien erfindungsgemäß hergestellten Zusammensetzung, enthalten.

Ein weiteres erfindungsgemäß außerordentlich bevorzugtes Verfahren ist dadurch gekennzeichnet, dass der polare Aktivator ausgewählt ist aus Propylencarbonat (4-Methyl-1,3-dioxolan-2-on), Wasser und Ethanol, sowie Mischungen hiervon, bevorzugt ausgewählt aus Propylencarbonat (4-Methyl-1,3-dioxolan-2-on).

Ein weiteres erfindungsgemäß außerordentlich bevorzugtes Verfahren ist dadurch gekennzeichnet, dass das Gewichtsverhältnis von Gesamtgewicht an Öl zum Gesamtgewicht an polarem Aktivator 35 - 100, bevorzugt 45 - 90, besonders bevorzugt 60 - 80, beträgt.

Im Sinne der vorliegenden Anmeldung bezieht sich der Ausdruck "Gesamtgewicht an Öl" auf das Gesamtgewicht aller enthaltenen kosmetischen Öle.

Die Homogenisierung erfolgt mit üblichen Homogenisiervorrichtungen, bevorzugt bei einer Scherrate von 500 - 2000 Umdrehungen pro Minute (Upm).

Ein weiteres erfindungsgemäß außerordentlich bevorzugtes Verfahren ist dadurch gekennzeichnet, dass der in Verfahrensschritt VI zugegebene kosmetische Wirkstoff mindestens eine Substanz umfasst, die unter Normalbedingungen fest ist und die in dem Öl bzw. der Ölmischung weder löslich noch quellbar ist.

Ein weiteres erfindungsgemäß außerordentlich bevorzugtes Verfahren ist dadurch gekennzeichnet, dass der unter Normalbedingungen feste, im Öl unlösliche und nicht quellbare kosmetische Wirkstoff ausgewählt ist aus mindestens einem schweißhemmenden Aluminiumsalz.

Bevorzugte schweißhemmende Aluminiumsalze sind ausgewählt aus den wasserlöslichen adstringierenden anorganischen und organischen Salzen von Aluminium. Alumosilicate und Zeolithe zählen erfindungsgemäß nicht zu den Antitranspirant-Wirkstoffen, können aber als absorbierende Bestandteile an der Schweißminderung beteiligt sein.

Erfindungsgemäß wird unter Wasserlöslichkeit eine Löslichkeit von wenigstens 3 Gew.-% bei 20 °C verstanden, das heißt, dass Mengen von wenigstens 3 g des Antitranspirant-Wirkstoffs in 97 g Wasser bei 20 °C löslich sind.

Besonders bevorzugte Antitranspirant-Wirkstoffe sind ausgewählt aus Aluminiumchlorhydrat, insbesondere Aluminiumchlorhydrat mit der allgemeinen Formel [Al₂(OH)₅Cl · 1-6 H₂O]ₙ, bevorzugt [Al₂(OH)₅Cl · 2-3 H₂O]ₙ, das in nicht-aktivierter oder in aktivierter (depolymerisierter) Form vorliegen kann, sowie Aluminiumchlorhydrat mit der allgemeinen Formel [Al₂(OH)₄Cl₂ · 1-6 H₂O]ₙ, bevorzugt [Al₂(OH)₄Cl₂ · 2-3 H₂O]ₙ, das in nicht-aktivierter oder in aktivierter (depolymerisierter) Form vorliegen kann.

Die Herstellung bevorzugter Antitranspirant-Wirkstoffe ist beispielsweise in US 3887692, US 3904741, US 4359456, GB 2048229 und GB 1347950 offenbart.

Weiterhin bevorzugt sind Aluminiumsesquichlorhydrat, Aluminiumdichlorhydrat, Aluminiumchlorhydrex-Propylenglykol (PG) oder Aluminiumchlorhydrex-Polyethylenglykol (PEG), Aluminium-Glycol-Komplexe, z. B. Aluminium-Propylenglycol-Komplexe, Aluminiumsesquichlorhydrex-PG oder Aluminiumsesquichlorhydrex-PEG, Aluminium-PG-dichlorhydrex oder Aluminium-PEGdichlorhydrex, Aluminiumhydroxid, weiterhin ausgewählt aus Kaliumaluminiumsulfat mit null bis 12 Teilen Kristallwasser (KAl(SO₄)₂ · 0 H₂O, KAl(SO₄)₂ · 1 H₂O, KAl(SO₄)₂ · 2 H₂O, KAl(SO₄)₂ · 3 H₂O, KAl(SO₄)₂ · 4 H₂O, KAl(SO₄)₂ · 5 H₂O, KAl(SO₄)₂ · 6 H₂O, KAl(SO₄)₂ · 7 H₂O, KAl(SO₄)₂ · 8 H₂O, KAl(SO₄)₂ · 9 H₂O, KAl(SO₄)₂ · 10 H₂O, KAl(SO₄)₂ · 11 H₂O KAl(SO₄)₂ · 12 H₂O = Alaun, teilhydratisierter Alaun bzw. gebrannter Alaun), Aluminiumundecylenoylcollagenaminosäure, Natriumaluminiumlactat + Aluminiumsulfat, Natriumaluminiumchlorhydroxylactat, Aluminiumbromhydrat, Aluminiumchlorid, den Aluminiumsalzen von Lipoaminosäuren, Aluminiumsulfat, Aluminiumlactat, Aluminiumchlorhydroxyallantoinat und Natrium-Aluminium-Chlorhydroxylactat. Erfindungsgemäß besonders bevorzugte Antitranspirant-Wirkstoffe sind ausgewählt aus so genannten "aktivierten" Aluminiumsalzen, die auch als Antitranspirant-Wirkstoffe "mit erhöhter Wirksamkeit (englisch: enhanced activity)" bezeichnet werden. Derartige Wirkstoffe sind im Stand der Technik bekannt und auch kommerziell erhältlich. Ihre Herstellung ist beispielsweise in GB 2048229, US 4775528 und US 6010688 offenbart. Aktivierte Aluminiumsalze werden in der Regel durch Wärmebehandlung einer relativ verdünnten Lösung des Salzes erzeugt (z.B. etwa 10 Gew.-% Salz), um dessen HPLC-Peak 4-zu-Peak 3-Flächenverhältnis zu vergrößern. Das aktivierte Salz kann anschließend zu einem Pulver getrocknet, insbesondere sprühgetrocknet werden. Neben der Sprühtrocknung ist z. B. auch die Walzentrocknung geeignet.

Aktivierte Aluminiumsalze haben typischerweise ein HPLC-Peak 4-zu-Peak 3-Flächenverhältnis von mindestens 0,4, bevorzugt mindestens 0,7, besonders bevorzugt mindestens 0,9, wobei mindestens 70% des Aluminiums diesen Peaks zuzuordnen sind.

Aktivierte Aluminiumsalze müssen nicht notwendigerweise als sprühgetrocknetes Pulver eingesetzt werden. Erfindungsgemäß ebenfalls bevorzugte schweißhemmende Wirkstoffe sind nichtwässrige Lösungen oder Solubilisate eines aktivierten schweißhemmenden Aluminiumsalzes, beispielsweise gemäß US 6010688, die durch den Zusatz einer wirksamen Menge eines mehrwertigen Alkohols, der 3 bis 6 Kohlenstoffatome und 3 bis 6 Hydroxyl-Gruppen, bevorzugt 1,2-Propylenglycol, Sorbit und Pentaerythrit, aufweist, gegen den Verlust der Aktivierung gegen den raschen Abbau des HPLC-Peak 4:Peak 3-Flächenverhältnisses des Salzes stabilisiert sind. Beispielsweise bevorzugt sind Zusammensetzungen, die in Gewichtsprozent (USP) enthalten: 18 - 45 Gew.-% eines aktivierten Aluminiumsalzes, 55 - 82 Gew.-% mindestens eines wasserfreien mehrwertigen Alkohols mit 3 bis 6 Kohlenstoffatomen und 3 bis 6 Hydroxyl-Gruppen, bevorzugt Propylenglycol, Butylenglycol, Diethylenglycol, Dipropylenglycol, Glycerin, Sorbit und Pentaerythrit, besonders bevorzugt Propylenglycol.

Besonders bevorzugt sind auch Komplexe aktivierter schweißhemmender Aluminiumsalze mit einem mehrwertigen Alkohol, die 20 - 50 Gew.-%, besonders bevorzugt 20 - 42 Gew.-%, aktiviertes schweißhemmendes Aluminiumsalz und 2 - 16 Gew.-% molekular gebundenes Wasser enthalten, wobei der Rest zu 100 Gew.-% mindestens ein mehrwertiger Alkohol mit 3 bis 6 Kohlenstoffatome und 3 bis 6 Hydroxyl-Gruppen ist. Propylenglycol, Propylenglycol/Sorbit-Mischungen und Propylenglycol/Pentaerythrit-Mischungen sind bevorzugte derartige Alkohole. Derartige erfindungsgemäß bevorzugte Komplexe eines aktivierten schweißhemmenden Aluminiumsalzes mit einem mehrwertigen Alkohol sind z. B. offenbart in US 5643558 und US 6245325.

Weitere bevorzugte schweißhemmende Wirkstoffe sind basische Calcium-Aluminiumsalze, wie sie beispielsweise in US 2571030 offenbart sind. Diese Salze werden durch Umsetzen von Calciumcarbonat mit Aluminiumchlorhydroxid oder Aluminiumchlorid und Aluminiumpulver oder durch Zusetzen von Calciumchlorid-Dihydrat zu Aluminiumchlorhydroxid hergestellt.

Weitere bevorzugte schweißhemmende Wirkstoffe sind aktivierte Aluminiumsalze, wie sie beispielsweise in US 6245325 oder US 6042816 offenbart sind, enthaltend 5 - 78 Gew.-% (USP) eines aktivierten schweißhemmenden Aluminiumsalzes, eine Aminosäure oder Hydroxyalkansäure in einer solchen Menge, um ein (Aminosäure oder Hydroxyalkansäure) zu Al - Gewichtsverhältnis von 2:1 - 1:20 und bevorzugt 1:1 bis 1:10 bereitzustellen, sowie ein wasserlösliches Calciumsalz in einer solchen Menge, um ein Ca:Al-Gewichtsverhältnis von 1:1 - 1:28 und bevorzugt 1:2 - 1:25 bereitzustellen. Besonders bevorzugte feste aktivierte schweißhemmende Salzzusammensetzungen, beispielsweise gemäß US 6245325 oder US 6042816, enthalten 48 - 78 Gew.-% (USP), bevorzugt 66 - 75 Gew.-% eines aktivierten Aluminiumsalzes und 1 - 16 Gew.-%, bevorzugt 4 - 13 Gew.-% molekular gebundenes Wasser (Hydratationswasser), weiterhin soviel wasserlösliches Calciumsalz, dass das Ca:Al-Gewichtsverhältnis 1:1 - 1:28, bevorzugt 1:2 - 1:25, beträgt und soviel Aminosäure, dass das Aminosäure zu Al - Gewichtsverhältnis 2:1 - 1:20, bevorzugt 1:1 - 1:10, beträgt.

Weitere besonders bevorzugte feste schweißhemmende aktivierte Salzzusammensetzungen, beispielsweise gemäß US 6245325 oder US 6042816, enthalten 48 - 78 Gew.-% (USP), bevorzugt 66 - 75 Gew.-% eines aktivierten Aluminiumsalzes und 1 - 16 Gew.-%, bevorzugt 4 - 13 Gew.-% molekular gebundenes Wasser (Hydratationswasser), weiterhin soviel wasserlösliches Calciumsalz, dass das Ca:Al-Gewichtsverhältnis 1:1 - 1:28, bevorzugt 1:2 - 1:25, beträgt und soviel Glycin, dass das Glycin zu Al - Gewichtsverhältnis 2:1 - 1:20, bevorzugt 1:1 - 1:10, beträgt.

Weitere besonders bevorzugte feste schweißhemmende aktivierte Salzzusammensetzungen, beispielsweise gemäß US 6245325 oder US 6042816, enthalten 48 - 78 Gew.-% (USP), bevorzugt 66 - 75 Gew.-% eines aktivierten Aluminiumsalzes und 1 - 16 Gew.-%, bevorzugt 4 - 13 Gew.-% molekular gebundenes Wasser, weiterhin soviel wasserlösliches Calciumsalz, dass das Ca:Al-Gewichtsverhältnis 1:1 - 1:28, bevorzugt 1:2 - 1:25, beträgt und soviel Hydroxyalkansäure, dass das Hydroxyalkansäure zu Al - Gewichtsverhältnis 2:1 - 1:20, bevorzugt 1:1 - 1:10, beträgt.

Für die Stabilisierung der schweißhemmenden Salze bevorzugte wasserlösliche Calciumsalze sind ausgewählt aus Calciumchlorid, Calciumbromid, Calciumnitrat, Calciumcitrat, Calciumformiat, Calciumacetat, Calciumgluconat, Calciumascorbat, Calciumlactat, Calciumglycinat, Calciumcarbonat, Calciumsulfat, Calciumhydroxid, sowie Mischungen davon.

Für die Stabilisierung der schweißhemmenden Salze bevorzugte Aminosäuren sind ausgewählt aus Glycin, Alanin, Leucin, Isoleucin, β-Alanin, Valin, Cystein, Serin, Tryptophan, Phenylalanin, Methionin, β-Amino-n-butansäure und y-Amino-n-butansäure und den Salzen davon, jeweils in der d-Form, der I-Form und der dl-Form; Glycin ist besonders bevorzugt.

Für die Stabilisierung der schweißhemmenden Salze bevorzugte Hydroxyalkansäuren sind ausgewählt aus Glycolsäure und Milchsäure.

Weitere bevorzugte schweißhemmende Wirkstoffe sind aktivierte Aluminiumsalze, wie sie beispielsweise in US 6902723 offenbart sind, enthaltend 5 - 78 Gew.-% (USP) eines aktivierten schweißhemmenden Aluminiumsalzes, eine Aminosäure oder Hydroxyalkansäure in einer solchen Menge, um ein (Aminosäure oder Hydroxyalkansäure) zu Al - Gewichtsverhältnis von 2:1 - 1:20 und bevorzugt 1:1 bis 1:10 bereitzustellen, sowie ein wasserlösliches Strontiumsalz in einer solchen Menge, um ein Sr:Al-Gewichtsverhältnis von 1:1 - 1:28 und bevorzugt 1:2 - 1:25 bereitzustellen.

Besonders bevorzugte feste schweißhemmende aktivierte Salzzusammensetzungen, beispielsweise gemäß US 6902723, enthalten 48 - 78 Gew.-% (USP), bevorzugt 66 - 75 Gew.-% eines aktivierten Aluminiumsalzes und 1 - 16 Gew.-%, bevorzugt 4 - 13 Gew.-% molekular gebundenes Wasser, weiterhin soviel wasserlösliches Strontiumsalz, dass das Sr:Al-Gewichtsverhältnis 1:1 - 1:28, bevorzugt 1:2 - 1:25, beträgt und soviel Aminosäure, dass das Aminosäure zu Al - Gewichtsverhältnis 2:1 - 1:20, bevorzugt 1:1 - 1:10, beträgt.

Weitere besonders bevorzugte feste schweißhemmende aktivierte Salzzusammensetzungen, beispielsweise gemäß US 6902723, enthalten 48 - 78 Gew.-% (USP), bevorzugt 66 - 75 Gew.-% eines aktivierten Aluminiumsalzes und 1 - 16 Gew.-%, bevorzugt 4 - 13 Gew.-% molekular gebundenes Wasser, weiterhin soviel wasserlösliches Strontiumsalz, dass das Sr:Al-Gewichtsverhältnis 1:1 - 1:28, bevorzugt 1:2 - 1:25, beträgt und soviel Glycin, dass das Glycin zu Al - Gewichtsverhältnis 2:1 - 1:20, bevorzugt 1:1 - 1:10, beträgt.

Weitere besonders bevorzugte feste schweißhemmende aktivierte Salzzusammensetzungen, beispielsweise gemäß US 6902723, enthalten 48 - 78 Gew.-% (USP), bevorzugt 66 - 75 Gew.-% eines aktivierten Aluminiumsalzes und 1 - 16 Gew.-%, bevorzugt 4 - 13 Gew.-% molekular gebundenes Wasser, weiterhin soviel wasserlösliches Strontiumsalz, dass das Sr:Al-Gewichtsverhältnis 1:1 - 1:28, bevorzugt 1:2 - 1:25, beträgt und soviel Hydroxyalkansäure, dass das Hydroxyalkansäure zu Al - Gewichtsverhältnis 2:1 - 1:20, bevorzugt 1:1 - 1:10, beträgt.

Weitere bevorzugte aktivierte Aluminiumsalze sind solche der allgemeinen Formel Al₂(OH)₆₋ₐXa, worin X Cl, Br, I oder NO₃ ist und "a" ein Wert von 0,3 bis 5, bevorzugt von 0,8 bis 2,5 und besonders bevorzugt 1 bis 2 ist, so dass das Molverhältnis von Al:X 0,9:1 bis 2,1:1 beträgt, wie sie beispielsweise in US 6074632 offenbart sind. Bei diesen Salzen ist im Allgemeinen etwas Hydratationswasser assoziativ gebunden, typischerweise 1 bis 6 Mol Wasser pro Mol Salz. Besonders bevorzugt ist Aluminiumchlorhydrat (d.h. X ist Cl in der vorgenannten Formel) und speziell 5/6-basisches Aluminiumchlorhydrat, worin "a" 1 beträgt, so dass das Molverhältnis von Aluminium zu Chlor 1,9:1 bis 2,1:1 beträgt.

Weitere bevorzugte schweißhemmende Wirkstoffe sind in US 6663854 und US 20040009133 offenbart.

Bevorzugte Aluminiumsalze weisen ein molares Metall-zu-Chlorid-Verhältnis von 0,9 - 1,3, bevorzugt 0,9 - 1,1, besonders bevorzugt 0,9 - 1,0, auf.

Besonders bevorzugte erfindungsgemäße Zusammensetzungen sind dadurch gekennzeichnet, dass der mindestens eine Antitranspirant-Wirkstoff in einer Menge 3 - 40 Gew.-%, bevorzugt 5 - 35 Gew.-%, besonders bevorzugt 10 - 30 Gew.-%, enthalten ist, bezogen auf das Gesamtgewicht der kristallwasserfreien Aktivsubstanz (USP) in der Gesamtzusammensetzung.

In einer besonders bevorzugten Ausführungsform enthält die Zusammensetzung ein adstringierendes Aluminiumsalz, insbesondere Aluminiumchlorohydrat, das beispielsweise pulverförmig als Micro Dry^{®} Ultrafine oder Superultrafine von Reheis, Microdry 323 von Summit, als Chlorhydrol^{®} sowie in aktivierter Form als Reach^{®} 501 von Reheis vertrieben wird. Unter der Bezeichnung Reach^{®} 301 wird ein Aluminiumsesquichlorohydrat von Reheis angeboten, das ebenfalls besonders bevorzugt ist. Besonders bevorzugt sind auch aktivierte Aluminiumchlorohydrate, die unter den Bezeichnungen Reach^{®} 101 und Reach^{®} 103, AACH-7171 von Reheis oder Summit erhältlich sind.

Da die schweißhemmenden Wirkstoffe in einem mit Wasser nicht mischbaren Träger suspendiert vorliegen, ist es aus Gründen der Produktstabilität bevorzugt, dass die Wirkstoffpartikel eine zahlenmittlere Partikelgröße von 0,1 - 200 µm, bevorzugt 1 - 150 µm, besonders bevorzugt 3 - 100 µm und außerordentlich bevorzugt 5 - 80 µm, aufweisen. Bevorzugte Wirkstoffpartikel weisen eine volumenmittlere Partikelgröße von 0,2 - 220 µm, bevorzugt 3 -160 µm, besonders bevorzugt 4 - 125 µm, weiterhin bevorzugt 5 - 120 µm und außerordentlich bevorzugt 10 - 80 µm, auf. Bevorzugte Aluminiumsalze weisen ein molares Metall-zu-Chlorid-Verhältnis von 1,9 -2,1, bzw. für Sesquichlorohydrate von 1,5:1-1,8:1 auf.

Besonders bevorzugte erfindungsgemäße Zusammensetzungen sind dadurch gekennzeichnet, dass der mindestens eine Antitranspirant-Wirkstoff in einer Menge von 3 - 40 Gew.-%, bevorzugt 5 - 35 Gew.-%, besonders bevorzugt 10 - 30 Gew.-%, enthalten ist, bezogen auf das Gesamtgewicht der kristallwasserfreien Aktivsubstanz (USP) in der treibmittelfreien Gesamtzusammensetzung.

In einer besonders bevorzugten Ausführungsform enthält die Zusammensetzung ein adstringierendes Aluminiumsalz, insbesondere Aluminiumchlorohydrat, besonders bevorzugt Aluminiumchlorohydrat mit einer kristallwasserfreien Aktivsubstanz (USP) von 72 - 88 Gew.-%, bezogen auf den Rohstoff tel quel. Bevorzugte nicht-aktivierte Aluminiumchlorohydrate sind beispielsweise pulverförmig als Micro Dry^{®}, Micro Dry^{®} Ultrafine oder Micro Dry^{®}-323 von Summit/Reheis, als Chlorhydrol^{®} (Pulver) sowie in aktivierter Form als Reach^{®} 101, Reach^{®} 103, Reach^{®} 501 von Reheis/Summit oder AACH-7171 von Summit vertrieben wird. Unter dem Namen Reach^{®} 301 wird ein Aluminiumsesquichlorohydrat von Reheis angeboten, das auch besonders bevorzugt ist. Neben dem oder an Stelle des in Verfahrensschritt VI zugegebenen Aluminiumsalzes können auch beliebige andere kosmetische Wirkstoffe in Verfahrensschritt VI zugegeben werden, insbesondere Deodorantwirkstoffe, Riechstoffe, Antioxidantien, Pflegestoffe, wie Vitamine und Proteine und andere. Das erfindungsgemäße Verfahren ist prinzipiell auch auf wasserhaltige kosmetische Zusammensetzungen bzw. Produkte anwendbar. Hierzu kann die verdickte Ölphase in Verfahrensschritt VI auch mit Wasser versetzt werden.

Besonders bevorzugte erfindungsgemäß hergestellte Zusammensetzungen enthalten weiterhin mindestens einen Riechstoff. Als Riechstoffe können Parfüme, Parfümöle oder Parfümölbestandteile eingesetzt werden. Parfümöle bzw. Duftstoffe können erfindungsgemäß einzelne Riechstoffverbindungen, z. B. die synthetischen Produkte vom Typ der Ester, Ether, Aldehyde, Ketone, Alkohole und Kohlenwasserstoffe sein. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat (DMBCA), Phenylethylacetat, Benzylacetat, Ethylmethylphenylglycinat, Allylcyclohexylpropionat, Styrallylpropionat, Benzylsalicylat, Cyclohexylsalicylat, Floramat, Melusat und Jasmecyclat. Zu den Ethern zählen beispielsweise Benzylethylether und Ambroxan , zu den Aldehyden z.B. die linearen Alkanale mit 8 - 18 C-Atomen, Citral, Citronellal, Citronellyloxy-acetaldehyd, Cyclamenaldehyd, Lilial und Bourgeonal, zu den Ketonen z.B. die Jonone, alpha-Isomethylionon und Methylcedrylketon, zu den Alkoholen Anethol, Citronellol, Eugenol, Geraniol, Linalool, Phenylethylalkohol und die Terpineole alpha-Terpineol, beta-Terpineol, gamma-Terpineol und delta-Terpineol, zu den Kohlenwasserstoffen gehören hauptsächlich die Terpene wie Limonen und Pinen. Bevorzugt werden jedoch Mischungen verschiedener Riechstoffe verwendet, die gemeinsam eine ansprechende Duftnote erzeugen. Die Riechstoffe können auch in verkapselter Form enthalten sein. Besonders bevorzugte erfindungsgemäße Zusammensetzungen enthalten mindestens einen Riechstoff in einer Gesamtmenge von 0,00001 bis 10 Gew.-%, bevorzugt 0,5 - 7 Gew.-%, außerordentlich bevorzugt 1 - 6 Gew.-%, jeweils bezogen auf das Gesamtgewicht der erfindungsgemäß hergestellten Zusammensetzung.

Ein weiteres erfindungsgemäß bevorzugtes Verfahren ist dadurch gekennzeichnet, dass der Verpackungsbehälter eine Aerosol-Abgabevorrichtung ist. Als erfindungsgemäß geeignete Aerosol-Abgabevorrichtung kommen übliche Spraydosen in Frage, die mit einem Ventil und einem Sprühkopf oder einer Sprühkappe ausgestattet sind. Als Spraydosen kommen Gefäße aus Metall (Aluminium, Weißblech, Zinn), geschütztem bzw. nicht-splitterndem Kunststoff oder aus Glas, das außen mit Kunststoff beschichtet ist, in Frage, bei deren Auswahl Druck- und Bruchfestigkeit, Korrosionsbeständigkeit, leichte Befüllbarkeit wie auch ästhetische Gesichtspunkte, Handlichkeit, Bedruckbarkeit etc. eine Rolle spielen. Spezielle Innenschutzlacke gewährleisten die Korrosionsbeständigkeit gegenüber der erfindungsgemäßen Zusammensetzung.

Ein weiteres erfindungsgemäß außerordentlich bevorzugtes Verfahren ist dadurch gekennzeichnet, dass

das Schichtsilikat das hydrophob modifizierte Schichtsilikat Disteardimonium Hectorite ist und
- der polare Aktivator Propylencarbonat (4-Methyl-1,3-dioxolan-2-on) ist und
- das Gewichtsverhältnis von Gesamtgewicht an Öl zum Gesamtgewicht an Disteardimonium Hectorite 12 - 45, bevorzugt 15 - 35, besonders bevorzugt 20 - 30, beträgt und
- das Gewichtsverhältnis von Gesamtgewicht an Öl zum Gesamtgewicht an Propylencarbonat (4-Methyl-1,3-dioxolan-2-on) 35 - 100, bevorzugt 45 - 90, besonders bevorzugt 60
- 80, beträgt und
- der in Verfahrensschritt VI zugegebene kosmetische Wirkstoff ein schweißhemmendes Aluminiumsalz umfasst und
- der Verpackungsbehälter eine Aerosol-Abgabevorrichtung ist.

Dieses außerordentlich bevorzugte Verfahren zur Herstellung eines kosmetischen Produkts, das eine kosmetische Zusammensetzung mit einer verdickten Ölphase und einen Verpackungsbehälter umfasst, weist also folgende Verfahrensschrittfolge auf:
I) Bereitstellen von mindestens einem Öl oder mindestens einer Ölmischung, das bzw. die unter Normalbedingungen flüssig ist,
II) Erwärmen des Öls bzw. der Ölmischung auf eine Temperatur von 30 - 55 °C, bevorzugt 30 - 35 °C,
III) Zugabe von soviel Disteardimonium Hectorite, dass das Gewichtsverhältnis von Gesamtgewicht an Öl zum Gesamtgewicht an Disteardimonium Hectorite 12 - 45, bevorzugt 15 - 35, besonders bevorzugt 20 - 30, beträgt, und von soviel Propylencarbonat (4-Methyl-1,3-dioxolan-2-on), dass das Gewichtsverhältnis von Gesamtgewicht an Öl zum Gesamtgewicht an Propylencarbonat (4-Methyl-1,3-dioxolan-2-on) 35 - 100, bevorzugt 45 - 90, besonders bevorzugt 60 - 80, beträgt,
IV) Homogenisieren, bevorzugt mit einer Scherrate von 500 bis 2000 Upm,
V) Abkühlen auf eine Temperatur im Bereich von 10 bis < 30 °C, bevorzugt im Bereich von 14 bis 22 °C,
VI) Zugabe von mindestens einem schweißhemmenden Aluminiumsalz und ggf. weiteren kosmetischen Wirk- und/oder Hilfsstoffen,
VII) Abfüllen in eine Aerosol-Abgabevorrichtung.

Die folgenden Beispiele sollen den Gegenstand der Erfindung verdeutlichen, ohne ihn hierauf zu beschränken.

Aus der folgenden Rezeptur wurde ein Antitranspirant-Spray nach dem herkömmlichen Verfahren hergestellt, das heißt, ohne Gelbildung in der Wärme, sondern bei Raumtemperatur.

| Bestandteile (INCI EU) | Gew.-%-Anteil |
|---|---|
| Butane | 73 |
| Propane | 11 |
| Isobutane | 1 |
| Cyclomethicone | 6,4 |
| Aluminum Chlorohydrate (USP) | 4,0 |
| Kristallwasser (gebunden, nicht frei) | 1,0 |
| Isopropyl Myristate | 1,0 |
| Disteardimonium Hectorite | 0,9 |
| Propylene Carbonate | 0,3 |
| Parfum | 1,4 |

Öle sind Cyclomethicone und Isopropylmyristat. Das Gewichtsverhältnis von Öl zu Disteardimonium Hectorite beträgt 7,4/0,9 = 8,22.

Das Gewichtsverhältnis von Öl zu Propylencarbonat beträgt 7,4/0,3 = 24,7.

Aus der folgenden Rezeptur wurde ein Antitranspirant-Spray nach dem erfindungsgemäßen Verfahren hergestellt.

| Bestandteile (INCI EU) | Gew.-%-Anteil |
|---|---|
| Butane | 73 |
| Propane | 10 |
| Isobutane | 2 |
| C10-13 Isoparaffin | 5,27 |
| Aluminum Chlorohydrate (USP) | 4,0 |
| Kristallwasser (gebunden, nicht frei) | 1,0 |
| Ethylhexyl Palmitate | 3,0 |
| Parfum | 1,0 |
| Disteardimonium Hectorite | 0,55 |
| Propylene Carbonate | 0,18 |

Öle sind C10-13 Isoparaffin und Ethylhexylpalmitat. Das Gewichtsverhältnis von Öl zu Disteardimonium Hectorite beträgt 8,27/0,55 = 15,04.

Das Gewichtsverhältnis von Öl zu Propylencarbonat beträgt 8,27/0,18 = 46.

Aus der folgenden Rezeptur wurde ebenfalls ein Antitranspirant-Spray nach dem erfindungsgemäßen Verfahren hergestellt.

| Bestandteile (INCI EU) | Gew.-%-Anteil |
|---|---|
| Butane | 71,0 |
| Isobutane | 2,1 |
| Propane | 10,4 |
| Isopentane | 1,0 |
| Pentane | 1,3 |
| Ethane | 0,2 |
| Cyclomethicone | 7,4 |
| Isopropyl Myristate | 1 |
| Parfum | 1,09 |
| Disteardimonium Hectorite | 0,38 |
| Kristallwasser (gebunden, nicht frei) | 1,0 |
| Aluminum Chlorohydrate | 4,0 |
| Propylene Carbonate | 0,13 |

Öle sind Cyclomethicone und Isopropylmyristat. Das Gewichtsverhältnis von Öl zu Disteardimonium Hectorite beträgt 8,4/0,38 = 22,1.

Das Gewichtsverhältnis von Öl zu Propylencarbonat beträgt 8,4/0,13 = 64,6.

Die Antitranspirant-Sprays, die nach dem erfindungsgemäßen Verfahren hergestellt wurden, erzielten die notwendige Viskosität mit deutlich weniger Disteardimonium Hectorite + Propylencarbonat im Verhältnis zum Gewicht des Öls als die herkömmlich hergestellten Sprays.

## Patentansprüche

1. Verfahren zur Herstellung eines kosmetischen Produkts, das eine kosmetische Zusammensetzung mit einer verdickten Ölphase und einen Verpackungsbehälter umfasst, **dadurch gekennzeichnet, dass** das Verfahren folgende Verfahrensschrittfolge aufweist:
I) Bereitstellen von mindestens einem Öl oder mindestens einer Ölmischung, das bzw. die unter Normalbedingungen (20 °C, 1013,25 mbar) flüssig ist,
II) Erwärmen des Öls bzw. der Ölmischung auf eine Temperatur von 30 - 55 °C, bevorzugt 30 - 35°C,
III) Zugabe von mindestens einem Schichtsilikat und mindestens einem polaren Aktivator,
IV) Homogenisieren, bevorzugt mit einer Scherrate von 500 - 2000 Umdrehungen pro Minute,
V) Abkühlen auf eine Temperatur im Bereich von 10 bis < 30 °C, bevorzugt im Bereich von 14 bis 22 °C,
VI) Zugabe von mindestens einem weiteren kosmetischen Wirk- und/oder Hilfsstoff,
VII) Abfüllen in einen Verpackungsbehälter.

2. Verfahren gemäß Anspruch 1, **dadurch gekennzeichnet, dass** das Schichtsilikat ausgewählt ist aus Hectorit, Bentonit, Montmorrillonit, Nontronit, Saponit, Sauconit, Beidellit, Allevardit, Illit, Halloysit, Attapulgit und/oder Sepiolit, sowie Mischungen hiervon.

3. Verfahren gemäß Anspruch 1 oder 2, **dadurch gekennzeichnet, dass** das Schichtsilikat mit mindestens einer organischen kationischen Substanz hydrophob modifiziert vorliegt.

4. Verfahren gemäß Anspruch 3, **dadurch gekennzeichnet, dass** das mindestens eine hydrophob modifizierte Schichtsilikat ausgewählt ist aus Disteardimonium Hectorite, Stearalkonium Hectorite, Stearalkonium Bentonite, Quaternium-18 Hectorite, Quaternium-18 Bentonite und Dihydrogenated Tallow Benzylmonium Hectorite, sowie Mischungen hiervon.

5. Verfahren gemäß Anspruch 1, 2, 3 oder 4, **dadurch gekennzeichnet, dass** der polare Aktivator ausgewählt ist aus Propylencarbonat (4-Methyl-1,3-dioxolan-2-on), Wasser und Ethanol, sowie Mischungen hiervon, bevorzugt ausgewählt aus Propylencarbonat (4-Methyl-1,3-dioxolan-2-on).

6. Verfahren gemäß Anspruch 1, 2, 3, 4 oder 5, **dadurch gekennzeichnet, dass** das Gewichtsverhältnis von Gesamtgewicht an Öl zum Gesamtgewicht an ggf. hydrophob modifiziertem Schichtsilikat 12 - 45, bevorzugt 15 - 35, besonders bevorzugt 20 - 30, beträgt.

7. Verfahren gemäß Anspruch 1, 2, 3, 4, 5 oder 6, **dadurch gekennzeichnet, dass** das Gewichtsverhältnis von Gesamtgewicht an Öl zum Gesamtgewicht an polarem Aktivator 35 - 100, bevorzugt 45 - 90, besonders bevorzugt 60 - 80, beträgt.

8. Verfahren gemäß Anspruch 1, 2, 3, 4, 5, 6 oder 7, **dadurch gekennzeichnet, dass** der in Verfahrensschritt VI zugegebene kosmetische Wirkstoff mindestens eine Substanz umfasst, die unter Normalbedingungen fest ist und die in dem Öl bzw. der Ölmischung weder löslich noch quellbar ist.

9. Verfahren gemäß Anspruch 1, 2, 3, 4, 5, 6, 7 oder 8, **dadurch gekennzeichnet, dass** der unter Normalbedingungen feste, im Öl unlösliche und nicht quellbare kosmetische Wirkstoff ausgewählt ist aus mindestens einem schweißhemmenden Aluminiumsalz.

10. Verfahren gemäß Anspruch 1, 2, 3, 4, 5, 6, 7, 8 oder 9, **dadurch gekennzeichnet, dass** der Verpackungsbehälter eine Aerosol-Abgabevorrichtung ist.

11. Verfahren gemäß Anspruch 1, 2, 3, 4, 5, 6, 7, 8, 9 oder 10, **dadurch gekennzeichnet, dass**
- das Schichtsilikat das hydrophob modifizierte Schichtsilikat Disteardimonium Hectorite ist und
- der polare Aktivator Propylencarbonat (4-Methyl-1,3-dioxolan-2-on) ist und
- das Gewichtsverhältnis von Gesamtgewicht an Öl zum Gesamtgewicht an Disteardimonium Hectorite 12 - 45, bevorzugt 15 - 35, besonders bevorzugt 20 - 30, beträgt und
- das Gewichtsverhältnis von Gesamtgewicht an Öl zum Gesamtgewicht an Propylencarbonat (4-Methyl-1,3-dioxolan-2-on) 35 - 100, bevorzugt 45 - 90, besonders bevorzugt 60
- 80, beträgt und
- der in Verfahrensschritt VI zugegebene kosmetische Wirkstoff ein schweißhemmendes Aluminiumsalz umfasst und
- der Verpackungsbehälter eine Aerosol-Abgabevorrichtung ist.

12. Verfahren gemäß Anspruch 1, 2, 3, 4, 5, 6, 7, 8, 9, 10 oder 11, **dadurch gekennzeichnet, dass** die Gesamtmenge an unter Normalbedingungen flüssigen kosmetischen Ölen 1 - 95 Gew.-%, bevorzugt 5 - 90 Gew.-%, besonders bevorzugt 30 - 75 Gew.-%, außerordentlich bevorzugt 50 - 60 Gew.-%, beträgt, wobei sich die Mengenangaben auf das Gewicht der kosmetischen Zusammensetzung beziehen, ohne das Gewicht des Treibmittels zu berücksichtigen.

13. Verfahren gemäß Anspruch 1, 2, 3, 4, 5, 6, 7, 8, 9, 10, 11 oder 12, **dadurch gekennzeichnet, dass** das Produkt ein Antitranspirant-Produkt ist und die Gesamtmenge an unter Normalbedingungen flüssigen kosmetischen Ölen 1 - 95 Gew.-%, bevorzugt 5 - 90 Gew.-%, besonders bevorzugt 10 - 85 Gew.-%, außerordentlich bevorzugt 20 - 80 Gew.-%, weiter bevorzugt 30 - 75 Gew.-%, ebenfalls sehr bevorzugt 50 - 70 Gew.-%, ebenfalls außerordentlich bevorzugt 54 - 64 Gew.-%, beträgt, wobei sich die Mengenangaben auf das Gewicht der kosmetischen Zusammensetzung beziehen, ohne das Gewicht des Treibmittels zu berücksichtigen.

14. Verfahren gemäß Anspruch 1, 2, 3, 4, 5, 6, 7, 8, 9, 10, 11, 12 oder 13, **dadurch gekennzeichnet, dass** das mindestens eine unter Normalbedingungen flüssige kosmetische Öl ausgewählt ist aus cyclischen Siliconölen mit der INCI-Bezeichnung Cyclomethicone, flüchtigen linearen Siliconölen, insbesondere flüchtigen linearen Siliconölen mit 2 - 10 Siloxan-Einheiten, C₈-C₁₆-Isoparaffinen, C₁₀-C₁₃-Isoparaffinmischungen, Estern der linearen oder verzweigten gesättigten oder ungesättigten Fettalkohole mit 2 - 30 Kohlenstoffatomen mit linearen oder verzweigten gesättigten oder ungesättigten Fettsäuren mit 2 - 30 Kohlenstoffatomen, die hydroxyliert sein können, Benzoesäureestern von linearen oder verzweigten C₈-C₂₂-Alkanolen, Triethylcitrat, verzweigten gesättigten oder ungesättigten Fettalkoholen mit 6 - 30 Kohlenstoffatomen, den Triglyceriden von linearen oder verzweigten, gesättigten oder ungesättigten, gegebenenfalls hydroxylierten C₈₋₃₀-Fettsäuren, sofern diese unter Normalbedingungen flüssig sind, Dicarbonsäureestern von linearen oder verzweigten C₂-C₁₀-Alkanolen, Anlagerungsprodukten von 1 bis 5 Propylenoxid-Einheiten an ein- oder mehrwertige C₈₋₂₂-Alkanole, Anlagerungsprodukten von mindestens 6 Ethylenoxid- und/oder Propylenoxid-Einheiten an ein- oder mehrwertige C₃₋₂₂-Alkanole, den symmetrischen, unsymmetrischen oder cyclischen Estern der Kohlensäure mit C₆-C₂₀-Alkoholen und den Estern von Dimeren ungesättigter C₁₂-C₂₂-Fettsäuren (Dimerfettsäuren) mit einwertigen linearen, verzweigten oder cyclischen C₂-C₁₈-Alkanolen oder mit mehrwertigen linearen oder verzweigten C₂-C₆-Alkanolen, sowie Mischungen hiervon.

15. Verfahren gemäß Anspruch 1, 2, 3, 4, 5, 6, 7, 8, 9, 10, 11, 12, 13 oder 14, **dadurch gekennzeichnet, dass** die treibmittelfreie Zusammensetzung mindestens ein hydrophob modifiziertes Schichtsilikat, das in Verfahrensschritt III zugesetzt wird, in einer Gesamtmenge von 0,5 - 10 Gew.-%, bevorzugt 1 - 7 Gew.-%, besonders bevorzugt 2 - 6 Gew.-%, außerordentlich bevorzugt 3 - 5 Gew.-%, enthält, jeweils bezogen auf das Gesamtgewicht der treibmittelfreien kosmetischen Zusammensetzung.
